# EUROPEAN PATENT APPLICATION

(11) **EP 4 729 107 A1**
(43) Date of publication of application: **22.04.2026**
(21) Application number: 24822838.9
(22) Date of filing: 14.06.2024
(51) Int. Cl.: A61M 25/10

(54) **HEART FAILURE THERAPEUTIC MAIN UNIT AND HEART FAILURE THERAPEUTIC INSTRUMENT**

(30) Priority: 16.06.2023 CN 202310721487
(71) Applicant: Pulnovo Medical Pte. Ltd., Singapore 189675 (SG)
(72) Inventor: ZHOU, Xin, Wuxi, Jiangsu 214191 (CN); CHEN, Mengxuan, Wuxi, Jiangsu 214191 (CN); TANG, Jian, Wuxi, Jiangsu 214191 (CN); ZHAO, Xinghai, Wuxi, Jiangsu 214191 (CN); ZHU, Rongjun, Wuxi, Jiangsu 214191 (CN)
(74) Representative: Mewburn Ellis LLP
(86) International application number: PCT/CN2024/099425
(87) International publication number: WO 2024/255891

(57) **Abstract**

The present disclosure provides a heart failure treatment host and a heart failure treatment instrument. The heart failure treatment host is used for being connected with the syringe in an assembled mode, the syringe comprises a needle cylinder and a piston rod located in the needle cylinder, the tail end of the piston rod is provided with at least one drag hook, the heart failure treatment host comprises an end cover used for limiting the syringe to an unlocking position and a locking position, and the locking position is reached when the syringe rotates around the axis of the syringe from the unlocking position; the rotating head is used for axially limiting the needle cylinder and can be driven by the needle cylinder to rotate; the push-pull rod is used for driving the piston rod to advance and retreat along the axis, a clamping groove is formed in the peripheral side of the front end of the push-pull rod, and the push-pull rod is provided with at least one avoiding notch extending from the front end face to be communicated with the clamping groove. The at least one drag hook is located in the clamping groove and is opposite to the at least one avoiding notch in a one-to-one correspondence mode, and under the condition that the injector is located at the locking position, the at least one drag hook is located in the clamping groove and is opposite to the at least one avoiding notch in an avoiding mode.

## Description

### BACKGROUND

Heart failure refers to a series of symptoms caused by insufficient blood perfusion due to heart dysfunction and inability to completely discharge blood from the heart. Heart failure is generally divided into acute heart failure and chronic heart failure.

Acute heart failure, the most common of which is acute left heart failure, refers to acute myocardial damage or increased cardiac load, which causes an acute decrease in cardiac output, an increase in pulmonary circulation pressure, and an increase in peripheral circulation resistance, which in turn causes pulmonary circulation congestion and acute pulmonary disease. Congestion, pulmonary edema, and clinical syndromes associated with tissue and organ hypoperfusion and cardiogenic shock may occur.

Chronic heart failure is divided into right heart failure and left heart failure. When the right heart fails, the ejection capacity of the right ventricle decreases, and more blood remains in the right heart, causing the pressure on the right heart to increase and blood to flow into the right heart with difficulty. Since the blood flowing into the right heart is the systemic circulation, there will be congestion in the systemic circulation and because the lower limbs are located in the lower parts of the body, congestion in the lower limbs is the most serious. When water enters the surrounding tissues from the blood vessels of the lower limbs, it causes edema of the lower limbs. When left heart failure occurs, because blood from the pulmonary circulation flows into the left heart, left atrial pressure and pulmonary venous pressure increase, which in turn causes pulmonary congestion and pulmonary edema.

In the related technology, according to the different conditions of acute heart failure, the initial treatment is oxygen inhalation, diuretics, and cardiotonic drugs are given intravenously. If the condition is still not relieved, vasoactive drugs are used to dilate and contract blood vessels. If the condition is severe, where the blood pressure continues to decrease or even cardiogenic shock, monitoring of hemodynamics and emergency non-pharmacological treatment is required.

Treatment modalities in the related art have certain limitations, for example, some patients respond well to intravenous diuretic treatment, but others do not. This is because when there is too much fluid in the body, most of the excess fluid is not actually found in the lumens of blood vessels, but in the spaces around cells, a tissue called the interstitium. To fully relieve congestion, excess fluid from the interstitium first needs to be moved into the blood vessels. In healthy humans, this function is accomplished through lymphatic drainage, which actively drains fluid into the large veins above the heart. However, in patients with acute heart failure, the pressure within the large veins can be very high, which can slow or even prevent the flow of lymph fluid into the large veins, hindering the process of decongestion. Research has found that incomplete diuresis (i.e. fluid retention) is a strong predictor of rehospitalization and death, with up to half of acute heart failure patients being discharged from hospital without being fully "diuretic", resulting in about 25% of patients being discharged from hospital within a month. This results in about half of patients being readmitted within six months.

The program of intermittent balloon filling and sealing of the superior and inferior vena cava blood can effectively reduce the pumping pressure of the heart, and can enhance the return of excess fluid in the interstitium into the lymphatic fluid and into the blood vessels, thereby reducing body fluid retention and improving the patient's postoperative cure. In the scheme of intermittent balloon filling and sealing of superior and inferior vena cava blood, the heart failure treatment host needs to cooperate with a disposable high-pressure syringe to intermittently fill the balloon catheter with fluid, pressurize it, and aspirate and release fluid according to the settings, thereby achieving intermittent balloon sealing. Intermittent balloon sealing blocks the superior vena cava and inferior vena cava to achieve the purpose of reducing the heart pumping pressure and reducing body fluid retention in heart failure.

### SUMMARY

Methods to improve the reliability of the connection between the heart failure treatment host and the syringe, thereby improving the effect of heart failure treatment, is a technical problem that needs to be solved urgently.

Embodiments of the present disclosure provide a heart failure treatment host and a heart failure treatment instrument to improve the reliability of the connection between the heart failure treatment host and the syringe, thereby improving the effect of heart failure treatment.

According to one aspect of the present disclosure, a heart failure treatment host is provided for assembly and connection with a syringe. The syringe includes a syringe and a piston rod located in the syringe, wherein the end of the piston rod has at least a retractor, the heart failure treatment host includes: an end cap for limiting the syringe in an unlocking position and a locking position, wherein the locking position is reached by the syringe rotating around its axis from the unlocking position; a rotating head, used to limit the axial position of the syringe and capable of being driven to rotate by the syringe; and a push-pull rod, used to drive the piston rod forward and backward along the axis, wherein the push-pull rod. There is a clamping groove on the peripheral side of the front end, and the push-pull rod has at least one escape notch extending from its front end surface to communicate with the clamping slot, wherein when the syringe is in the unlocking position, the at least one hook is located in the slot and faces the at least one avoidance notch in a one-to-one correspondence. When the syringe is in the locking position, the at least one hook is located in the slot and avoids contact with the at least one avoidance notch. At least one avoidance gap is opposite.

In some embodiments, the slot is an annular slot.

In some embodiments, the at least one hook is an interference fit with the slot.

In some embodiments, the at least one draw hook is two draw hooks arranged oppositely, and the at least one escape notch is two escape notches arranged oppositely.

In some embodiments, the locked position is reached by rotating the syringe 90° clockwise or 90° counterclockwise about its axis from the unlocked position.

In some embodiments, the syringe has a flange, and the periphery of the flange has a first positioning protrusion; the end cap has a first blocking part, a second blocking part and a syringe for the syringe to pass through. The first through hole, wherein when the syringe is in the unlocking position, the first positioning protrusion is adjacent to the first blocking portion, and when the syringe is in the locking position, the first positioning protrusion is adjacent to the second blocking portion.

In some embodiments, the end cap has a flange groove for positioning the flange, and the first blocking part, the second blocking part and the first through hole are provided in the flange groove.

In some embodiments, the heart failure treatment host further includes: a rotating head base, the rotating head is rotatably provided on the rotating head base, wherein the end circumferential side of the syringe has two oppositely arranged second positioning protrusions, the rotating head is provided with a second through hole for the needle barrel to pass through, and two positioning grooves corresponding to the two second positioning protrusions.

In some embodiments, the heart failure treatment host is to be assembled and connected with two syringes arranged side by side, wherein the two syringes rotate in the same direction from the unlocking position to the locking position.

According to another aspect of the present disclosure, a heart failure treatment device is provided, including: the heart failure treatment host of the aforementioned aspect; a syringe assembled and connected to the heart failure treatment host; and a catheter assembled and connected to the syringe.

These and other aspects of the disclosure will be apparent from and elucidated with reference to the embodiments described hereinafter.

### BRIEF DESCRIPTION OF THE DRAWINGS

**Figure 1** is a schematic three-dimensional structural diagram of the assembly and connection of the heart failure treatment host and the syringe according to some embodiments of the present disclosure;
**Figure 2** is a schematic front view of the assembly and connection of the heart failure treatment host and the syringe according to some embodiments of the present disclosure;
**Figure 3** is a schematic diagram of the three-dimensional structure of a syringe in some embodiments of the present disclosure;
**Figure 4** is a partially disassembled structural diagram of a heart failure treatment host and a syringe according to some embodiments of the present disclosure; and
**Figure 5** is a schematic diagram of the cross-sectional structure cut along the A-A direction as in Figure 1.

Further details, features and advantages of the present disclosure are disclosed in the following description of exemplary embodiments in conjunction with the accompanying figures.

### DETAILED DESCRIPTION

It should be understood that in this specification, the terms "center", "longitudinal", "transverse", "length", "width", "thickness", "upper", "lower", "front", "rear" , "Left", "Right", "Vertical", "Horizontal", "Top", "Bottom", "Inside", "Outside", "Clockwise", "Counterclockwise", "Axis", " the orientation or positional relationship or dimensions indicated by "radial", "circumferential", etc. are based on the orientation or positional relationship or dimensions shown in the drawings. These terms are used only for convenience of description and do not indicate or imply the device or device to which they are referred. Elements must have a specific orientation, be constructed and operate in a specific orientation and therefore should not be construed as limiting the scope of the disclosure.

Furthermore, the terms "first", "second", "third", etc. are used for descriptive purposes only and cannot be understood as indicating or implying relative importance or implicitly indicating the quantity of indicated technical features. Thus, features defined as "first", "second", and "third" may explicitly or implicitly include one or more of these features. In the description of the present disclosure, "plurality" means two or more than two, unless otherwise expressly and specifically limited.

In this disclosure, unless otherwise explicitly stated and limited, the terms "installation", "connection", "connection", "fixing" and other terms should be understood in a broad sense. For example, it can be a fixed connection or a detachable connection, or integrated. It can be a mechanical connection, an electrical connection, or a communication. It can be a direct connection, or an indirect connection through an intermediate medium, or an internal connection between two elements or an interaction between two elements . For those of ordinary skill in the art, the specific meanings of the above terms in this disclosure can be understood according to specific circumstances.

In this disclosure, unless otherwise expressly stated and limited, a first feature "on" or "below" a second feature may include the first and second features in direct contact, or may include the first and second features. Not in direct contact but through additional characteristic contact between them. Furthermore, the terms "above", "over" and "on" a first feature on a second feature include the first feature being directly above and diagonally above the second feature, or simply mean that the first feature is higher in level than the second feature. "Below", "under" and "beneath" the first feature is the second feature includes the first feature being directly below and diagonally below the second feature, or simply means that the first feature is less horizontally than the second feature.

Explanation of reference symbols: 100-Heart failure treatment host; 200-syringe; 21-needle barrel ; 22-piston rod; 221-retractor; 211-flange; 212-first positioning protruding part; 213-second positioning protruding part; 13-end cover; 131-first blocking part; 132-second blocking part; 133-first through hole; 134-flange groove; 14-rotating head base; 15-rotating head; 151-second through hole; 152-positioning groove; 16-push-pull rod; 161-card slot; 162-avoidance gap.

The present disclosure relates to the technical field of medical equipment, and in particular to a heart failure treatment host and a heart failure treatment instrument. According to one or more embodiments of the present disclosure, it is not only convenient to disassemble and assemble the syringe, but also can improve the reliability of the connection between the heart failure treatment host and the syringe, thereby improving the effect of heart failure treatment.

The heart failure treatment host needs to cooperate with a disposable high-pressure syringe to fill and release the balloon. In related technologies, when the heart failure treatment host is assembled or disassembled from a disposable high-pressure syringe, disassembly and assembly are often difficult because the rubber piston at the end of the piston rod in the disposable high-pressure syringe does not rotate in place. In addition, the rubber piston does not rotate in place. It will also lead to poor contact with the push-pull rod, so that it cannot be accurately driven by the push-pull rod, resulting in weakened balloon filling and release effects, and poor heart failure treatment effects.

Embodiments of the present disclosure provide a heart failure treatment host and a heart failure treatment instrument to improve the reliability of the connection between the heart failure treatment host and the syringe, thereby improving the effect of heart failure treatment.

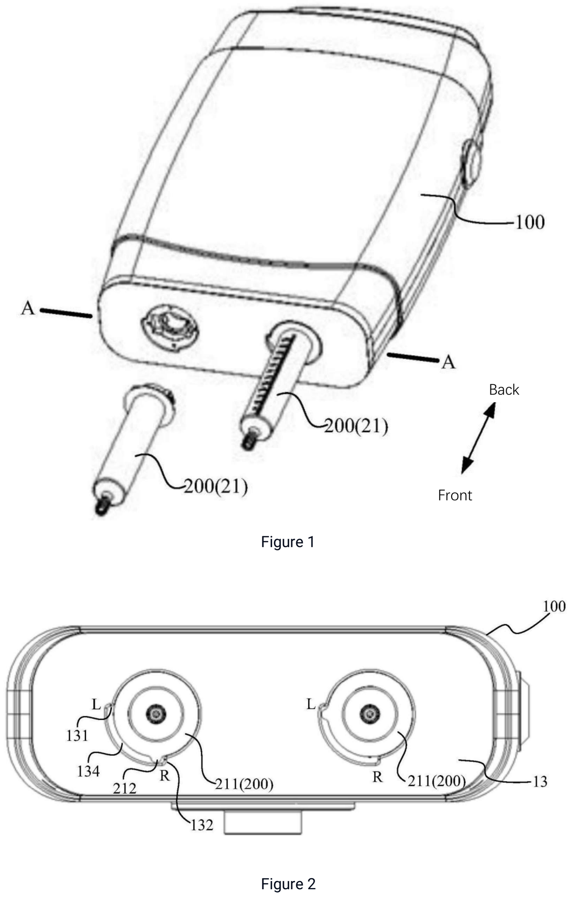

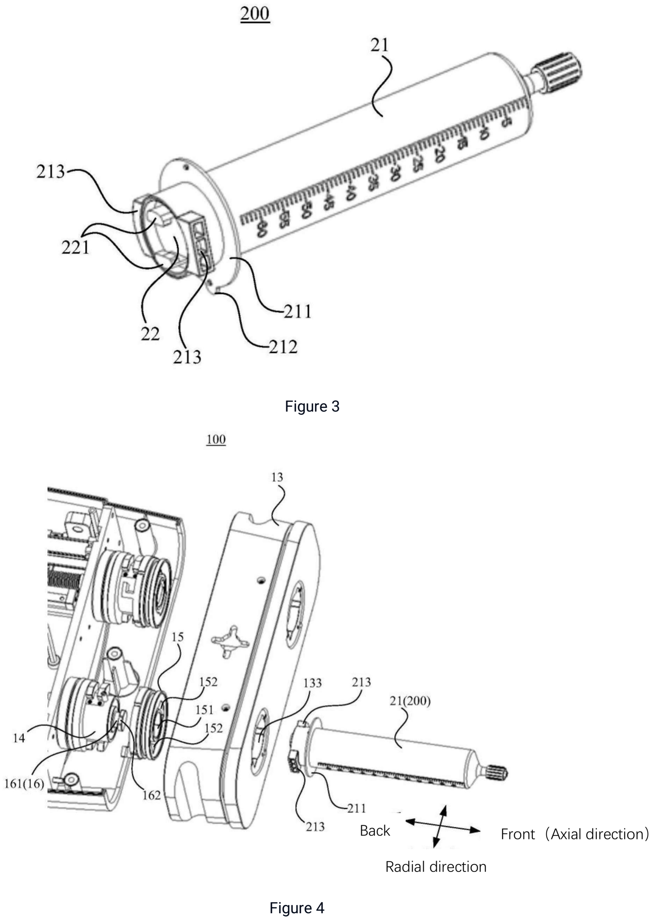

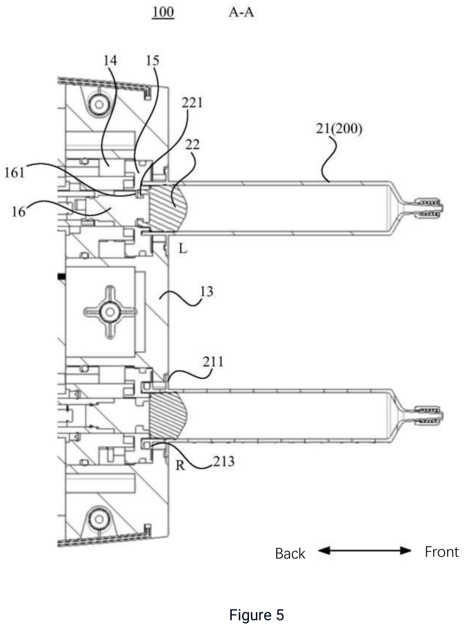

Like Figure 1, Figure 2, and Figure 3, as shown, the heart failure treatment host 100 provided by some embodiments of the present disclosure is used for assembly and connection with the syringe 200. The syringe 200 includes a needle barrel 21 and a piston rod 22 located therein, wherein the end of the piston rod 22 can be configured as a rubber piston with at least one hook 221. Like Figure 4 and Figure 5, as shown in the figures, the structure of the heart failure treatment host 100 includes an end cap 13, a rotating head 15, and a push-pull rod 16 .

Like Figure 2, as shown, the end cap 13 is used to limit the syringe 200 at the unlocking position R and the locking position L. The locking position L is reached by rotating the syringe 200 from the unlocking position R around its axis. The rotating head 15 is used to limit the axial position of the needle barrel 21 and can be driven and rotated by the needle barrel 21. The push-pull rod 16 is used to drive the piston rod 22 forward and backward along the axis. See Figure 4 as shown in the figures, the push-pull rod 16 has a latching groove 161 on its front end peripheral side, and the push-pull rod 16 has at least one escape notch 162 extending from its front end surface to communicate with the latching groove 161.

Like Figure 2 and Figure 5, as shown, when the syringe 200 is in the unlocking position R, the at least one hook 221 is located in the slot 161 and faces the at least one escape notch 162 in one-to-one correspondence. At this time, the push-pull rod 16 and the piston rod 22 are in the axial direction. Without interference, the at least one hook 221 can enter the slot 161 through the at least one avoidance gap 162. As in Figure 2 and Figure 5, two syringes 200 are shown, respectively in the unlocked position R and the locked position L.

Like Figure 2 and Figure 5, as shown in the figures, when the syringe 200 is in the locking position L, the at least one hook 221 is located in the slot 161 and avoids facing the at least one escape notch 162. At this time, the push-pull rod 16 and the piston rod 22 are axially opposite to each other. Therefore, the push-pull rod 16 can drive the piston rod 22 forward and backward along the axis.

Like Figure 4 and Figure 5 as shown, when the heart failure treatment host 100 of the embodiment of the present disclosure is assembled and connected with the syringe 200, the syringe 200 is set in the unlocking position R. At this time, the push-pull rod 16 and the piston rod 22 are also unlocked from each other in the axial direction, and the syringe 200 is rotated to the locking position L. At this time, the push-pull rod 16 and the piston rod 22 are also locked with each other in the axial direction. Therefore, when the push-pull rod 16 drives the piston rod 22 forward and backward, the push-pull rod 16 can reliably interact with the piston rod 22. When locked, the rubber piston at the end of the piston rod 22 can be accurately driven by the push-pull rod 16. The design of the heart failure treatment host 100 of the present disclosure is not only convenient for disassembly and assembly with the syringe 200, but also can improve the reliability of the connection between the heart failure treatment host 100 and the syringe 200, thereby improving the effect of heart failure treatment.

In embodiments of the present disclosure, the side of the component closer to the needle (not shown in the figure) of the syringe 200 is defined as "front", and the side of the component further away from the needle (not shown in the figure) of the syringe 200 is defined as "rear" and the direction is as shown in the figures.

Like Figure 1 and Figure 2, as shown, in some embodiments, the heart failure treatment host 100 is used to be assembled and connected with two syringes 200 arranged side by side, so that one heart failure treatment host 100 and two syringes 200 can be used to achieve intermittent balloon filling and occlusion of the blood from superior and inferior vena cava. Under this design, the above-mentioned structures of the syringe 200 are respectively arranged into two groups. For example, the heart failure treatment host 100 includes two rotating heads 15 and two push-pull rods 16.

Like Figure 2, as shown, in some embodiments, the locked position L is reached by rotating the syringe 200 90° clockwise or 90° counterclockwise about its axis from the unlocked position R. Choosing an appropriate rotation stroke can provide a certain feel for the operation and can further improve the reliability of the locking between the push-pull rod 16 and the piston rod 22.

In some embodiments, the rotation direction of the two syringes 200 from the unlocking position R to the locking position L is the same. In this way, the two syringes 200 are installed in the same manner, which can further improve the convenience of assembly and facilitate the rapid installation of the syringes 200.

### Disassembly and Assembly

Like Figure 4 as shown in the embodiment of the present disclosure, the front end of the push-pull rod 16 has a slot 161 on the peripheral side, thereby forming a stud structure on the front side of the slot 161. The at least one avoidance notch 162 extends from the front end surface of the push-pull rod 16 to the card slot 161, that is, the at least one escape notch 162 can be formed on the column head structure.

In some embodiments of the present disclosure, there is no limit to the number of hooks 221 provided on the rubber piston at the end of the piston rod 22. According to the design size of the hook 221 in the circumferential direction, the strength of the hook 221, the required connection strength design between the piston rod 22 and the push-pull rod 16 may be adjusted. The number of the hooks 221 can be designed to be one, two or more. In some embodiments of the present disclosure, as in Figure 3 as shown, two hooks 221 are provided at the end of the piston rod 22, and the two hooks 221 are arranged oppositely in the circumferential direction. Correspondingly, two opposite avoidance notches 162 are provided at the front end of the push-pull rod 16. Such a design ensures that the piston rod 22 is balanced in force in the radial direction, which is more conducive to the accurate driving of the rubber piston of the piston rod 22 by the push-pull rod 16.

In some embodiments, the slot 161 is an annular slot. In other embodiments of the present disclosure, the clamping groove 161 may also be designed as multiple segments distributed along the circumferential direction according to the number and rotational stroke of the hooks 221 at the end of the piston rod 22 , which is not specifically limited in the present disclosure.

In some embodiments of the present disclosure, at least one hook 221 on the rubber piston has an interference fit with the slot 161. The hook 221 is made of rubber and has an interference fit with the slot 161. In this way, there is a certain amount of friction between the two, and relative rotation between the piston rod 22 and the push-pull rod 16 is difficult to occur in the absence of external force or a non-artificial small external force. This can further improve the accuracy of assembly and the reliability of connection between the two.

In some embodiments of the present disclosure, the end cap 13 is used to limit the syringe 200 in the unlocking position R and the locking position L. Like Figure 2, Figure 3, and Figure 4 As shown in the figure, the needle barrel 21 has a flange 211, and the flange 211 has a first positioning protrusion 212 on its periphery. The flange 211 is one end away from the end of the needle barrel 21. The end cap 13 has a first blocking part 131, a second blocking part 132, and a first through hole 133 for the needle barrel 21 to pass through. When the syringe 200 is in the unlocking position R, the first positioning protrusion 212 is adjacent to the first blocking part 131, and when the syringe 200 is in the locking position L, the first positioning protrusion 212 is adjacent to the second blocking part 132. The specific structural forms of the first blocking part 131 and the second blocking part 132 are not limited, as long as they can block and stop the syringe 200 in the unlocking position R and the locking position L.

In some embodiments of the present disclosure, as Figure 2, the end cover 13 has a flange groove 134 for positioning the flange 211, and the first blocking part 131, the second blocking part 132 and the first through hole 133 are provided in the flange groove 134. In this way, the flange 211 can only rotate at a certain angle around the axis in the flange groove 134 without moving in the radial direction. This design is beneficial to improving the precision of assembly.

In some embodiments, as in Figure 4 and Figure 5, the heart failure treatment main unit, or host, 100 also includes a rotating head base 14, and the rotating head 15 is rotatably provided on the rotating head base 14. The needle barrel 21 has two second positioning protrusions 213 oppositely disposed on the peripheral side of the distal end. The rotating head 15 is provided with a second through hole 151 for the needle barrel 21 to pass through, and a second through hole 151 for the needle barrel 21 to pass through. Two positioning grooves 152 are provided correspondingly. When the two second positioning protrusions 213 are located in the two positioning grooves 152 in one-to-one correspondence, the rotating head 15 can rotate along with the rotation of the needle barrel 21.

The structural design of the above embodiment is only for part of the structure of the heart failure treatment host 100, and the present disclosure does not specifically limit the structural design of other parts of the product.

In some embodiments, the present disclosure also provides a heart failure treatment device, including the heart failure treatment host 100 of any of the foregoing embodiments, a syringe 200 assembled and connected to the heart failure treatment host 100, and a catheter assembled and connected to the syringe 200.

Since the heart failure treatment host 100 and the syringe 200 can be reliably connected, the therapeutic effect of the heart failure treatment device is improved. In addition, the disassembly and assembly operations of the heart failure treatment host 100 and the syringe 200 are also more convenient.

This specification provides many different embodiments or examples that can be used to implement the disclosure. It should be understood that these various embodiments or examples are purely exemplary and are not intended to limit the scope of the present disclosure in any way. Those skilled in the art can think of various changes or substitutions based on the disclosure of the present disclosure, and these should be covered by the protection scope of the present disclosure. Therefore, the protection scope of the present disclosure should be subject to the protection scope defined by the appended claims.

## Claims

1. A heart failure treatment unit or host, for assembly and connection with a syringe,
the syringe comprising:
a syringe barrel; and
a piston rod located within the syringe barrel, wherein the piston rod has at least one hook at its end; and
the heart failure treatment host comprising:
an end cap configured to limit the syringe to an unlocked position and a locked position, wherein the locked position is reached by the syringe rotating from the unlocked position about its axis;
a rotating head, configured to axially limit the syringe and capable of being driven to rotate by the syringe; and
a push-pull rod configured to drive the piston rod forward and backward along the axis, wherein the front end peripheral side of the push-pull rod has a groove, and the push-pull rod has at least one clearance notch extending from its front end face to communicate with the groove,
wherein when the syringe is in the unlocked position, the at least one hook is located in the groove and corresponds to the at least one clearance notch, and when the syringe is in the locked position, the at least one hook is located in the groove and avoids being opposite to the at least one clearance notch.

2. The heart failure treatment host according to claim 1, wherein the slot is an annular slot.

3. The heart failure treatment host according to claim 1, wherein the at least one hook is interference-fitted with the slot.

4. The heart failure treatment host according to claim 1, wherein the at least one hook is two hooks arranged opposite each other, and the at least one clearance notch is two clearance notches arranged opposite each other.

5. The heart failure treatment host according to claim 1, wherein the locking position is reached by rotating the syringe 90° clockwise or 90° counterclockwise around its axis from the unlocking position.

6. The heart failure treatment host according to claim 1, wherein the syringe has a flange, and the periphery of the flange has a first positioning protrusion; the end cap has a first blocking portion, a second blocking portion, and a first through hole through which the syringe passes, wherein, when the syringe is in the unlocked position, the first positioning protrusion is adjacent to the first blocking portion, and when the syringe is in the locked position, the first positioning protrusion is adjacent to the second blocking portion.

7. The heart failure treatment host according to claim 6, wherein the end cap has a flange groove for positioning the flange, and the first blocking part, the second blocking part and the first through hole are provided in the flange groove.

8. The heart failure treatment host according to claim 1, further comprising a rotating head base, wherein the rotating head is rotatably disposed on the rotating head base, wherein the end periphery of the syringe has two opposing second positioning protrusions, and the rotating head is provided with a second through hole for the syringe to pass through and two positioning grooves that are correspondingly matched with the two second positioning protrusions.

9. The heart failure treatment host according to any one of claims 1 to 8, wherein the heart failure treatment host is configured to be assembled and connect with two syringes arranged side by side, wherein the two syringes rotate in the same direction from the unlocked position to the locked position.

10. A heart failure treatment device, comprising:
the heart failure treatment host according to any one of claims 1 to 9;
a syringe assembled and connected to the heart failure treatment host;
a catheter assembled and connected to the syringe.
